# EUROPEAN PATENT APPLICATION

(11) **EP 4 666 994 A1**
(43) Date of publication of application: **24.12.2025**
(21) Application number: 24183020.7
(22) Date of filing: 19.06.2024
(51) Int. Cl.: A61K 6/35, A61K 6/887, A61K 6/30

(54) **DENTURE ADHESIVE**

(71) Applicant: Haleon UK IP Limited, Weybridge, Surrey KT13 0NY (GB)
(72) Inventor: The designation of the inventor has not yet been filed
(74) Representative: Haleon Patent Department

(57) **Abstract**

The present invention relates to denture adhesive compositions having a reduced viscosity facilitating ease of application and use, particularly for older adults or consumers having limited hand movement or strength. Despite a reduced viscosity, the compositions exhibit hold (adhesion over time) and other characteristics comparable with commercially available formulations.

## Description

### FIELD OF THE INVENTION

The present invention relates to denture adhesive compositions having a reduced viscosity facilitating ease of application, use and removal, particularly for older adults or consumers having limited hand movement or strength. Despite a reduced viscosity, the compositions exhibit hold (adhesion over time) and other characteristics comparable with commercially available formulations.

### BACKGROUND TO THE INVENTION

Full or partial dentures are forms of dental appliance intended to be worn in the mouth as substitutes serving as a replacement for some or all of the teeth usually found in the oral cavity. When using dentures, it is common to use an adhesive to help adhere the dental appliance to a surface of the oral cavity, such as the palate or a gum ridge. Such denture adhesives, also referred to as fixatives, secure or temporarily fix dentures within the mouth, improving retention of the dentures, whilst also being releasable so that the denture wearer may remove the dentures for cleaning and maintenance. Denture adhesives can provide an improvement in the denture wearing experience even if the denture fits well and may also be beneficial in situations where dentures might not fit perfectly, for example as a consequence of natural shrinkage and changes in the gum or oral mucosa over time. Prior to placement of the denture in the oral cavity, the denture adhesive is applied to the denture plate surface. In use the compositions fill the interstices between the dentures and surfaces of the oral cavity providing uniform contact.

As such, these compositions need to fulfill a number of, often competing, criteria in order to function effectively: they should develop a high degree of tack upon contact with saliva so that the dentures are held in place as soon as they are seated in the mouth, possess sufficient cohesive strength to withstand the stresses of mastication which act to dislodge the denture and provide a seal against ingress of food residue beneath the denture. Further, the organoleptic properties of the denture adhesive composition, such as mouthfeel and consistency should be acceptable to the user and the composition should not be too messy or sticky to apply.

Despite considerable effort to develop denture adhesive compositions over the years, there remains a need for further denture adhesives offering improvements in user experience.

### SUMMARY OF THE INVENTION

The Inventors have developed compositions with desirable adhesive properties, particularly for use with dental appliances such as dentures.

In a First Aspect, there is provided a denture adhesive composition comprising from about 18 to about 25 %w/w of medium viscosity mineral oil and an aloe component, wherein the composition has a viscosity of from 300,000-2,000,000 centipoise.

Particularly, there is provided a denture adhesive composition comprising: (a) from about 18 to about 25 %w/w of medium viscosity mineral oil, (b) an *aloe* component, (c) an alkyl vinyl ether-maleic acid copolymer or salt thereof, (d) petrolatum and (e) carboxymethylcellulose, wherein the composition has a viscosity of from 300,000-2,000,000 centipoise.

More particularly, there is provided a denture adhesive composition comprising: (a) from about 18 to about 25 %w/w of medium viscosity mineral oil, (b) an *aloe* component, (c) from about 28 to about 32 percent by weight (%w/w) of an alkyl vinyl ether-maleic acid copolymer or salt thereof, (d) from about 18 to about 26 %w/w of petrolatum and (e) from about 23 to about 25 %w/w of carboxymethylcellulose, wherein the composition has a viscosity of from 300,000-2,000,000 centipoise.

Particularly denture adhesive compositions of the Invention comprise from about 0.02 to about 2.5 %w/w of the aloe component. More particularly, the aloe component is selected from the group consisting of an aloe extract, a powdered aloe extract, an aloe extract in oil or combination thereof.

In some embodiments of the Invention, the aloe component is an aloe extract in oil, such as a plant seed oil comprising an aloe extract. Particularly, the aloe component is an aloe extract in oil, more particularly an aloe extract in plant seed oil, yet more particularly an aloe extract in *Helianthus annuus* (*sunflower*) seed oil. In certain embodiments, the aloe component is an aloe extract in *Helianthus annuus* seed oil, particularly from about 1% to about 5% of an aloe extract in from about 99% to about 95% of *Helianthus annuus* seed oil. Particularly the aloe component comprises from about 1% to about 5% of *Aloe barbadensis* leaf extract, for example obtained from the dried juice leaves of the aloe plant, in from about 99% to about 95% of *Helianthus annuus*seed oil. More particularly, the aloe component comprises from about 1% to about 5% of *aloe barbadensis* leaf extract in from about 99% to about 95% of *Helianthus annuus* seed oil.

In some embodiments, the aloe component further comprises a powdered aloe extract or an aloe flavour agent. In other embodiments, the aloe component further comprises a powdered aloe extract and an aloe flavour agent. The aloe component may comprise 0.1% w/w of a powdered aloe extract and/or 0.1% w/w of an aloe flavour agent.

In certain embodiments, the powdered aloe extract is aloe juice leaf powder obtained from the dried juice leaves of the *aloe barbadensis* plant.

Particularly the alkyl vinyl ether-maleic acid copolymer or salt thereof is a poly(methylvinylether/maleic acid) salt. More particularly the alkyl vinyl ether-maleic copolymer or salt thereof is a poly(methylvinylether/maleic acid) Sodium-Calcium Mixed Partial Salt.

Particularly the petrolatum has a cone penetration consistency value of from about 100 to about 200 (mm/10 at 25oC). More particularly the petrolatum has a cone penetration consistency value of from about 130 to about 195 (mm/10 at 25oC).

Particularly, the carboxymethylcellulose has a viscosity of about 1000 to about 2800 centipoise (cP) (measured in a 1% solution at 25°C). More particularly, the carboxymethylcellulose consists of fine particles of which at least 80% pass through a 200 U.S. mesh sieve (equivalent to a 74 µm mesh sieve). Preferably, the carboxymethylcellulose is sodium carboxymethylcellulose, for example sodium carboxymethylcellulose optionally having a degree of substitution (DS) of from 0.65 to 0.95, particularly about 0.7.

Particularly, following preparation, the denture adhesive compositions of the Invention have a viscosity of at least 300,000 centipoise (cP), such as at least 400,000 cP. Particularly, following preparation, the denture adhesive compositions of the Invention have a viscosity of no more than 2,500,000 cP, more particularly no more than 2,000,000 cP and yet more particularly no more than 1,000,000 cP. Particularly, following preparation, the denture adhesive compositions of the Invention have a viscosity of from 400,000 to 2,500,000 centipoise (cP), more particularly of from 400,000 to 2,000,000 cP or yet more particularly of from 400,000 to 1,000,000 cP. Particularly the compositions have a pH of from 4.0 to 10, more particularly a pH of from 5.0 to 8.0 and still yet more particularly a pH of from 6.0 to 8.0. More particularly the denture adhesive compositions of the Invention have (i) a viscosity of from 400,000 to 2,000,000 centipoise (cP) and/or (ii) a pH of from 4.0 to 10, more particularly a pH of from 5.0 to 8.0. Preferably the denture adhesive composition has (i) a viscosity of from 400,000-2,000,000 centipoise ((cP) measured at 25°C ± 1°C) and/or (ii) a pH of from 4.0 to 10, particularly a pH of from 5.0 to 8.0.

In certain preferred embodiments, the denture adhesive composition comprises or consists essentially of: (a) from 28-32% w/w of poly(methylvinylether/maleic acid) Sodium-Calcium Mixed Partial Salt; (b) from 23-25% w/w of sodium carboxymethylcellulose; (c) from 18-25% w/w of medium viscosity mineral oil; (d) from 18%-26% w/w of petrolatum; (e) 1.5-2.5% w/w of *Helianthus annuus* (sunflower) seed oil comprising from 1% to 5% of an *aloe barbadensis* extract; and optionally (f) 0.1% w/w of an aloe flavour agent or a powdered aloe extract.

Particularly such compositions have a viscosity of from 400,000 to 2,000,000 centipoise (cP) and a pH of from 5.0 to 8.0. More particularly such compositions have a viscosity of from 400,000 to 1,000,000 centipoise (cP) and a pH of from 6.0 to 8.0.

More particularly, when applied to a support surface (carrier) the denture adhesive composition exhibits work of adhesion values of at least 5 J/m², for up to 20 dislodgement cycles in an Instron based in vitro adhesion model.

In particular embodiments, the denture adhesive composition of the Invention are free of silica, titanium dioxide, sodium alginate, zinc or combinations thereof.

In a Second Aspect, there is provided a denture adhesive composition according to the First Aspect for use in a method of adhering a dental appliance to a surface, particularly a surface in the oral cavity. There is also provided a method of adhering a dental appliance to a surface, particularly a surface in the oral cavity, using a denture adhesive composition according to the First Aspect. There is also provided a denture adhesive composition according to the First Aspect for use in a method of improving food seal of a dental appliance. There is also provided a denture adhesive composition according to the First Aspect for use in a method of preventing or reducing ingress of food particles between a dental appliance and a surface in the oral cavity. Particularly, the methods may comprise applying a composition of the First Aspect to a dental appliance, the oral cavity, or both, and applying the dental appliance to a surface of the oral cavity, such as the palate or a gum ridge.

### DESCRIPTION OF FIGURES

**FIGURE 1****:** Bar chart of Instron work of adhesion values for formulations 12, 13 and 15 compared to a commercially available control (Poligrip Max Hold & Seal).
**FIGURE 2****:** Graph of Instron work of adhesion values over time for formulations 12 (■), 13 (Δ) and 15 (X) compared to a commercially available control (◆, Poligrip Max Hold & Seal).
**FIGURE 3****:** Bar chart of Instron work of adhesion values for formulations 16 and 17 compared to a commercially available control (Poligrip Max Hold & Seal).
**FIGURE 4****:** Graph of Instron work of adhesion values over time for formulations 16 (▲) and 17 (■) compared to a commercially available control (•, Poligrip Max Hold & Seal).

### DETAILED DESCRIPTION OF THE INVENTION

The present inventors have discovered improved denture adhesive compositions having a reduced viscosity facilitating ease of application and use, particularly for older adults or consumers having limited hand movement or strength. The compositions comprise an aloe component for improved sensorial effects. Thus, compositions of the Invention provide an immediate, strong and long-lasting hold whilst also offering improvements in consumer perception of the compositions, for example, providing an improved application experience, for example improved spreading and softness, and/or reducing the amount of adhesive oozing out of the denture and into the mouth over time. Surprisingly, rheology measurements demonstrate that the compositions of the Invention are softer and hydrate in a more controlled and uniform way. Further, compositions of the Invention have an adhesion profile that facilitates removal at the end of the day without leaving residue in the mouth.

The denture adhesive compositions of the Invention generally comprise from about 18 to about 25 %w/w of medium viscosity mineral oil and an aloe component, such compositions having a viscosity of from 300,000-2,000,000 centipoise. In particular, the compositions may comprise, consist or consist essentially of: (a) from about 18 to about 25 %w/w of medium viscosity mineral oil, (b) an *aloe* component, (c) an alkyl vinyl ether-maleic acid copolymer or salt thereof, (d) petrolatum and (e) carboxymethylcellulose, wherein the composition has a viscosity of from 300,000-2,000,000 centipoise.

As used herein, the term "dental appliance" refers to dentures or partial dentures, artificial teeth, removable orthodontic bridges and denture plates, both upper and lower types, orthodontic retainers and appliances, protective mouthguards, nightguards to prevent bruxism and/or Temporomandibular joint (TMJ) disorder, and the like. The term "denture" is used to refer to a subset of dental appliances which includes dentures or partial dentures, such as upper and lower dentures and combinations thereof, including orthodontic bridges, artificial teeth, denture plates and the like.

The terms "denture adhesive composition" and "denture adhesive" are terms used in the art and refer to a composition for use with a denture to enhance retention, stability and function.

For the avoidance of doubt, reference to "% by weight" (%w/w) means by weight of the total composition, the amount of each ingredient being selected so that total ingredients in the composition sum to 100 percent by weight.

### Aloe Component

Particularly denture adhesive compositions of the Invention comprise from about 0.02 to about 5.0 %w/w of an aloe component. For example, from about 0.1 to about 2.5% w/w, from about 1.5 to about 2.5% w/w, from about 1.75% to about 2.5% w/w, from about 1.75% to about 2.0% w/w or from about 2.0% to about 2.5% w/w of an aloe component, such as about 0.1%, 0.25%, 0.5%, 0.75%, 1.0%, 1.25%, 1.5%, 1.75%, 2.0%, 2.25% or 2.5%.

The term "aloe" refers to the genus of flowering succulent plants of the Asphodelaceae family of which representative species include Aloe arborescens, Aloe barbadensis, Aloe cremnophila, Aloe ferox and Aloe saponaria. A preferred species of aloe is Aloe vera, also known as Aloe barbadensis Miller.

The term "aloe component" refers to a preparation obtained from the leaves of an Aloe plant by chemical or mechanical action, for example by pressure, distillation or evaporation. Aloe leaves have three layers: (1) an inner clear gel composed of water, glucomannans, amino acids, lipids, sterols and vitamins; (2) an intermediate layer (surrounding the gel) of latex that contains anthraquinones and glycosides; and (3) an outer skin, often referred to as the rind, that comprises the xylem and phloem and also has a protective function. Non-limiting examples of aloe components include the juice of the whole leaf of the plant, inner leaf gel preparations (where the outside skin and thorns of the leaf have been stripped away to reveal the gel-like "fillet") and dehydrated compositions such as powder preparations of the whole leaf or gel fillet. The term also includes concentrated gel preparations (for example, made by partially dehydrating aloe gel or fully dehydrating and reconstituting aloe gel) and oil preparations (for example, prepared by blending/macerating aloe gel in a carrier oil such as deodorised sunflower oil).

Particularly the aloe component is decolourised. Particularly the aloe component is substantially free of anthraquinones. Particularly the aloe component is decolourised and substantially free of anthraquinones. Aloin, also known as barbaloin, is the major anthraquinone of aloe. Therefore, preferably the aloe component is substantially free of aloin. The term "substantially free of aloin" refers to a content of less than 1 ppm of aloin. Thus, in certain preferred embodiments, the aloe component is decolourised and substantially free of aloin. Particularly, the aloe component is decolourised and comprises less than 1 ppm of aloin.

The aloe component may be selected from the group consisting of a powdered aloe extract, a concentrated gel preparation or an aloe extract in oil or combinations thereof, for example a combination of powdered aloe extract and an aloe extract in oil. Suitable powdered aloe extracts include powdered aloe vera gel 100: 1 or 200:1. However, the use of powdered extracts is less preferred as they may add a 'gritty' texture to the composition that is less favoured by consumers.

Preferably, the aloe component is an aloe extract in oil, for example an aloe extract in mineral oil or an aloe extract in plant oil.

The term "plant oil" refers to an oil extracted, or otherwise obtained from, either directly or indirectly, plant sources. Plant oils are known to the skilled person and may include, but are not limited to, safflower (*Carthamus tinctorius*) oil, sunflower (*Helianthus annuus*) oil, peanut (*Arachis hypogaea*) oil, walnut (*Juglans regia*) oil, almond (*Prunus dulcis*) oil, avocado (*persea americana*) oil, olive (*Olea europaea*) oil, corn (*Zea mays*) oil, soybean (*Glycine max*) oil, coconut (*Cocos nucifera*) oil, palm (*Elaeisguineensis*) oil and the like. Other plant oils are known to those having ordinary skill in the art.

The plant oil may be a refined oil. The term "refined oil" refers to a plant oil that has undergone a refining process to remove unwanted constituents, for example to remove proteins that may be responsible for allergic reactions. Suitable processes for refining plant oils are well known in the art. The plant oil may also be deodorized to reduce or eliminate any unwanted odors or scents.

In certain preferred embodiments, the aloe extract is an aloe extract in *Helianthus annuus* (sunflower) seed oil. The term "seed oil" refers to a plant oil extracted, or otherwise obtained from, either directly or indirectly, the seeds of a plant.

In certain embodiments, the aloe extract in oil comprises from 1% to 5% of an aloe extract, particularly from 1% to 5% of an *aloe barbadensisextract.* In more particular embodiments, the aloe extract in oil is a preparation of *Helianthus annuus* seed oil comprising from 1% to 5% of an *aloe barbadensisextract.* In other more particular embodiments, the aloe extract in oil is a preparation of mineral oil comprising from 1% to 5% of an *aloe barbadensisextract.* In this case, and for the avoidance of doubt, the mineral oil content in the preparation of mineral oil comprising from 1% to 5% of an *aloe barbadensisextract* is considered to be part of the aloe component, not the medium viscosity mineral oil component.

### Medium Viscosity Mineral Oil

Compositions of the Invention comprise a medium viscosity mineral oil.

Mineral oils (CAS No. 8042-47-5) are liquid mixtures of hydrocarbons, being generally colourless, odourless and tasteless transparent, oily liquids that are considered GRAS (Generally Regarded as Safe). Mineral oils may be characterised in terms of their kinematic viscosity. Kinematic viscosity may be measured at 40°C by means of a calibrated glass capillary viscometer as set out in the ASTM Standard Test Method for Kinematic Viscosity of Transparent and Opaque Liquids D445 (DOI: 10.1520/D0445-21E01). A light mineral oil will generally have a kinematic viscosity of about 34 cSt or less at 40°C. Medium and heavy mineral oils will generally have a kinematic viscosity ranging from about 35 cSt up to about 240 cSt at 40°C.

Particularly, the oil is a medium viscosity mineral oil, more particularly a medium viscosity mineral oil having a kinematic velocity of from about 35 cSt to about 90 cSt at 40°C and yet more particularly a medium viscosity mineral oil having a kinematic velocity of from about 63 cSt to about 80 cSt at 40°C.

In certain embodiments, the medium viscosity mineral oil may have a density of 860 to 870kg/m³ measured at 20°C using a digital density meter, such as a DMA 46 densitometer (from Anton Paar) or equivalent, calibrated with purified water.

Suitable commercially available medium viscosity mineral oils include PIONIER 2071P or equivalent.

Particularly, the compositions comprise from about 18% to about 25% by weight (%w/w) of an oil component, preferably medium viscosity mineral oil for example, 18%, 19%, 20%, 21%, 22%, 23%, 24% or 25% by weight of medium viscosity mineral oil, particularly from about 19% to about 22% by weight of medium viscosity mineral oil, for example 19.1%, 19.2%, 19.3%, 19.4%, 19.5%, 19.6%, 19.7%, 19.8%, 19.9%, 20.0%, 20.1%, 20.2%, 20.3%, 20.4%, 20.5%, 20.6%, 20.7%, 20.8%, 20.9%, 21.0%, 21.1%, 21.2%, 21.3%, 21.4%, 21.5%, 21.6%, 21.7%, 21.8%, 21.9% or 22.0%.

### Alkyl vinyl ether-maleic acid copolymer

The denture adhesive compositions of the Invention comprise an alkyl vinyl ether-maleic acid (AVE/MA) copolymer or salt thereof, such as a mixed salt, a partial salt or a mixed partial salt. As used herein, the term "mixed salt" refers to salts wherein at least two different cations are mixed on the same polymer with each other or with other salts. The term, "partial salt" refers to salts of polymers where less than 100 percent of the acid groups are reacted.

Particularly, the denture adhesive compositions comprise an alkyl vinyl ether-maleic acid copolymer partial salt. More particularly, the denture adhesive compositions comprise an alkyl vinyl ether-maleic acid copolymer mixed partial salt.

The alkyl vinyl ether-maleic acid copolymer comprises at least one cationic salt selected from the group consisting of strontium, zinc, iron, magnesium, calcium and sodium. Preferably, the alkyl vinyl ether-maleic acid copolymer contains at least one cationic salt selected from the group consisting of calcium and sodium. Thus, in some embodiments the AVE/MA copolymer contains a cationic salt selected from calcium and sodium or a combination thereof. Preferably, the at least one cationic salt is a combination of calcium and sodium cations.

The metal cations may be present on the alkyl vinyl ether-maleic acid copolymer in an amount to provide from about 10.0 percent to about 20.0 percent of metal cations, such as about 12.0% to about 16.0%, for example about 12.0%, about 12.5%, about 13.0%, about 13.5%, about 14.0%, about 14.5%, about 15.0% or about 15.5%.

Particularly, the alkyl vinyl ether-maleic acid copolymer comprises or consists essentially of from about 1.0 percent to about 15.0 percent calcium cations and from about 0.5 percent to about 5.0 percent sodium cations. More particularly, the alkyl vinyl ether-maleic acid copolymer comprises or consists essentially of from about 9.0 percent to about 15.0 percent calcium cations and from about 1.0 percent to about 3.0 percent sodium cations. Preferably the alkyl vinyl ether-maleic acid copolymer is free of zinc cations.

Particularly, the denture adhesive composition comprises from about 28 to about 34 percent by weight of the alkyl vinyl ether-maleic acid copolymer or salt thereof, for example about 28%, 29%, 30%, 31%, 32%, 33% or 34% w/w of the alkyl vinyl ether-maleic acid copolymer or salt thereof. More particularly, the denture adhesive composition comprises from 28% to about 32% w/w of the alkyl vinyl ether-maleic acid copolymer or salt thereof. In other embodiments, the denture adhesive composition comprises about 34 percent by weight of the alkyl vinyl ether-maleic acid copolymer or salt thereof. from 28-32% w/w of poly(methylvinylether/maleic acid) Sodium-Calcium Mixed Partial Salt

Preferably, the alkyl vinyl ether-maleic acid copolymer or salt thereof is a poly(methylvinylether/maleic acid) salt. More preferably, the alkyl vinyl ether-maleic acid copolymer or salt thereof is a poly(methylvinylether/maleic acid) Sodium-Calcium Mixed Partial Salt.

Thus, in certain embodiments, the denture adhesive composition comprises from about 28 to about 34 percent by weight of poly(methylvinylether/maleic acid) Sodium-Calcium Mixed Partial Salt, such as about 28%, 29%, 30%, 31%, 32%, 33% or 34% w/w of poly(methylvinylether/maleic acid) Sodium-Calcium Mixed Partial Salt. In more particular embodiments, the denture adhesive composition comprises from about 28 to about 32 percent by weight of poly(methylvinylether/maleic acid) Sodium-Calcium Mixed Partial Salt, such as about 28%, 29%, 30%, 31% or 32% w/w of poly(methylvinylether/maleic acid) Sodium-Calcium Mixed Partial Salt.

Examples of suitable commercially available alkyl vinyl ether-maleic acid copolymers are sold under the trade name GANTREZ (ISP Investments LLC) available from Thermo Fisher or Ashland. These include the the GANTREZ MS form such as GANTREZ MS- 955 (a mixed sodium and calcium salt of methyl vinyl ether and maleic anhydride copolymer).

### Carboxymethylcellulose

Denture adhesive compositions of the Invention comprise carboxymethylcellulose (CMC), a cellulose derivative. Particularly, the compositions comprise from about 23% to about 25% by weight of CMC, for example, about 20%, 21%, 22%, 23% or 24% by weight of CMC, particularly 24% by weight of CMC. Preferably the CMC is food or pharmaceutical grade CMC.

Particularly, the CMC is provided in the form of a powder prior to use in a composition of the Invention, more particularly in the form of a fine powder, more particularly in the form of a fine powder of which at least 99.9% of the particles pass through a 60 U.S. mesh sieve, more particularly at least 90% of the particles pass through an 80 U.S. mesh sieve, yet more particularly at least 80% of the particles pass through a 100 U.S. mesh sieve and still yet more particularly at least 80% of the particles pass through a 200 U.S. mesh sieve

Particularly, the CMC has a viscosity of from about 800 to about 3000 centipoise (cP), preferably a viscosity of from about 1000 to about 2800 cP (measured in a 1% solution at 25°C).

Particularly, the CMC has a degree of substitution (DS) of from about 0.65 to about 0.95, such as from about 0.65 to about 0.85, preferably about 0.7.

Preferably the CMC is sodium carboxymethylcellulose. Particularly the CMC is sodium carboxymethylcellulose having a sodium content of from 6.5% to 9.5% such as from 7.0% to about 9.2%, more particularly of from about 7.0% to about 8.9%. Preferably the sodium carboxymethylcellulose is food or pharmaceutical grade.

Examples of commercially available CMCs useful in compositions of the Invention include the 7H products available from Aqualon such as 7H3SXF or 7H3SF and products available from Dupont such as WALOCEL CRT 2000, WALOCEL CRT 10000 or WALOCEL CRT 15000, such as WALOCEL CRT 15000PPA (S) Sodium. In certain embodiments, the sodium CMC is sodium CMC 7H3SXF, 7H3SF, 7H3SXF PH, 7H3SF PH, WALOCEL CRT 15000, WALOCEL CRT 15000PPA (S) Sodium or equivalents.

In certain embodiments, the sodium carboxymethylcellulose is food or pharmaceutical grade CMC having a viscosity of from about 1000 to about 2800 cP (measured in a 1% solution at 25°C), a degree of substitution of from about 0.65 to about 0.95, a sodium content of from 6.5% to 9.5% and an average molecular weight of about 725,000.

### Petrolatum

The term "petrolatum" is a term of the art used to refer to a semi-solid mixture of hydrocarbons. The viscosity of petrolatum may be determined using the ASTM Standard Test Method for Cone Penetration of Petrolatum (D937-07) to determine the penetration of petrolatum as an empirical measure of consistency. The petrolatum may have a cone penetration consistency value (mm/10 at 25°C) of from about 10 to about 500, particularly from about 25 to about 300 and more particularly from about 50 to about 250. Preferably, the petrolatum has a cone penetration consistency value of from about 100 to about 200, more preferably from about 130 to about 195 (mm/10 at 25°C).

Particularly, denture adhesive compositions of the Invention may comprise from about 18 to about 26 percent by weight of petrolatum, for example about 18%, 19%, 20%, 21%, 22%, 23%, 24%, 25% or 26% by weight of petrolatum. More particularly from about 22 to about 24 percent by weight of petrolatum, for example 22.0%, 22.25%, 22.5%, 22.75%, 23.0%, 23.25%, 23.5%, 23.75% or 24.0%, by weight of petrolatum.

### Other Components

Denture adhesive compositions may comprise at least one sensory component. A "sensory component" refers to a component that may be used to impart a flavour, fragrance, colour and/or sensation (such as cooling, warming or tingling) to the composition. Other than imparting the compositions with organoleptic properties that are desirable for consumers, generally the sensory component is not an active component, in other words it does not play an active role in adhesion, hold time or cushioning. Particularly, the sensory component is at least one flavour, fragrance and/or colouring agent. Sensory components are known in the art and may be natural or artificial (synthetic).

Compositions of the invention may comprise from about 0.01% to about 5% by weight of a sensory component, particularly from about 0.01% to about 2% by weight of a sensory component.

Particularly, the sensory component is a flavouring agent. Flavouring agents well known in the denture adhesive art may be added to the compositions of the present invention. These flavouring agents include, but are not limited to, synthetic flavour oils and/or oils derived from plants, leaves, flowers, fruits and so forth, and combinations thereof. Representative flavour oils include aloe, spearmint oil, cinnamon oil, oil of wintergreen (methylsalicylate) and peppermint oils. Also useful are artificial, natural or synthetic fruit flavours such as citrus oil including lemon, orange, grape, lime, and grapefruit, and fruit essences including apple, strawberry, cherry, pineapple, and so forth. The flavouring agent may be a liquid, spray dried, encapsulated, or absorbed on a carrier, and mixtures thereof. The amount of flavouring agent utilized varies depending on such factors as flavour type, adhesive composition and strength desired. In certain embodiments, the denture adhesive composition comprises from about 0.01 percent to about 3.0 percent by weight of the flavouring agent. In certain embodiments, the denture adhesive composition comprises from about 0.01 percent to about 2.0 percent by weight of the flavouring agent. In certain embodiments, the denture adhesive composition comprises from about 0.05 percent to 2.0 percent by weight of the flavouring agent, particularly about 0.1% by weight of a flavouring agent.

In particular embodiments, the composition comprises from about 0.05% to about 2% of an aloe flavouring agent, particularly about 0.1% by weight of an aloe flavouring agent. The term, "aloe flavouring agent" is intended to refer to a flavouring agent that imparts an aloe flavour, as its dominant flavour, to a consumer. Suitable aloe flavouring agents are known in the art.

The denture adhesive compositions may also include the use of sweeteners well known in the art. The sweetening agent may be selected from a wide range of materials including water-soluble agents, water-soluble artificial sweeteners, and dipeptide-based sweeteners, including mixtures thereof. Representative sweeteners include, but are not limited to, (a) sugar alcohols such as sorbitol, xylitol, mannitol, maltitol, hydrogenated starch hydrolysate, and mixtures thereof; (b) water-soluble artificial sweeteners such as the soluble saccharin salts, i.e., sodium or calcium saccharin salts, cyclamate salts, Acesulfame-K, sucralose, and the like, and the free acid form of saccharin; (c) dipeptide based sweeteners such as L-aspartyl-L-phenylalanine methyl ester, and the like; and (d) natural sweeteners such as stevia. In certain embodiments, the denture adhesive composition comprises from about 0.001 percent to about 5.0 percent by weight of the sweetening agent.

The colourants useful in the present invention include the pigments and dyes suitable for food, drug and cosmetic applications. These colourants are known as FD&C (Food, Drugs and Cosmetics) and D&C (Drugs & Cosmetics) dyes and lakes. Illustrative examples include, but are not limited to, D&C red#30 Aluminium Lake Paste, D&C Red #7 Calcium Lake Paste, Erythrosine Lake Paste, indigo dye, known as FD&C Blue No. 2, which is the disodium salt of 5,5 -indigotindi-sulfonic acid; FD&C Green No. 1, comprising a triphenylmethylene dye and is the monosodium salt of the 4-[4-N-ethyl-p-sulfobenzylamino)diphenylmethylene]-[I-(N-ethyl-N-P-sulfobenzyl)-2,5-cyclohexadienimine]. In some embodiments, the colorant is FD&C Red No. 3. In some embodiments, the denture adhesive composition comprises from about 0.001 percent to about 0.2 percent by weight of colourant.

In certain embodiments, the denture adhesive composition is free of fragrance and/or colouring agents, i.e. it does not comprise any fragrance and/or colouring agents (beyond the intrinsic properties of components (a), (b) (c) and (d).

In certain embodiments, the denture adhesive composition is free of flavour, fragrance and/or colouring agents, i.e. it does not comprise flavour, fragrance and/or colouring agents (beyond the intrinsic properties of components (a), (b) (c) and (d).

Adhesive properties of the compositions of the Invention may be assessed by means of an Instron based *in vitro* adhesion model. In this model, two surfaces are joined together by a layer of denture adhesive and the force required to separate the surfaces is measured. This data is used to generate a force-displacement curve from which the maximum force of detachment and total work of adhesion (area under curve or W_{AUC}) may be determined.

Particularly when applied to a support surface (carrier) the denture adhesive exhibits total work of adhesion values of 300 J/m² or higher, in an Instron based *in vitro* adhesion model.

Denture adhesive compositions of the Invention may be dispensed by the consumer from a multidose package, pump or tube. Alternatively, the denture adhesive compositions of the Invention may be pre-dispensed, in single dose packages, for example on a non-adhesive self-supporting layer that may be peeled off to enable the adhesive to be applied.

To facilitate dispensing, when measured following preparation of the composition, the denture adhesive compositions of the Invention may have a viscosity of from 300,000-2,500,000 centipoise (cP).

Particularly, following preparation, the denture adhesive compositions of the Invention have a viscosity of at least 300,000 centipoise (cP) or more particularly at least 400,000 centipoise (cP). Particularly, following preparation, the denture adhesive compositions of the Invention have a viscosity of no more than 2,500,000 cP, more particularly no more than 2,000,000 cP and yet more particularly no more than 1,000,000 cP, such as no more than 500,000 cP. Particularly, following preparation, the denture adhesive compositions of the Invention have a viscosity of from 300,000 to 2,500,000 centipoise (cP), more particularly of from 400,000 to 2,000,000 cP, yet more particularly of from 400,000 to 1,000,000 cP. For the avoidance of doubt, viscosity may be measured within two months of preparation of the composition, more particularly within one month of preparation of the composition, such as a viscosity of from 300,000 to 2,500,000 cP when measured within one month of preparation of the composition or 400,000 to 1,000,000 cP when measured within one month of preparation of the composition. Preferably, viscosity is measured at 25°C ± 1°C.

Compositions of the invention preferably have a pH within the range of from 4.0 to 10.0, such as a pH within the range of from 5.0 to 8.0 or a pH within the range of from 6.0 to 8.0, preferably a pH of from about 6.0 to about 7.5, such as a pH of from about 6.2 to about 7.5 or a pH of from 6.5 to 7.5.

Particularly the denture adhesive compositions of the Invention have (i) a viscosity of from 300,000 to 2,000,000 centipoise (cP) and/or (ii) a pH of from 4.0 to 10, more particularly a pH of from 5.0 to 8.0. Preferably the denture adhesive composition has (i) a viscosity of from 400,000-2,000,000 centipoise ((cP) measured at 25°C ± 1°C) and/or (ii) a pH of from 4.0 to 10, particularly a pH of from 5.0 to 8.0, more particularly a pH of from 6.0 to 8.0. In certain embodiments, the denture adhesive composition has (i) a viscosity of from 300,000-2,000,000 centipoise, particularly from 400,000-2,000,000, yet more particularly from 400,000-1,000,000 cP still yet more particularly from 400,000 to 500,000 cP (measured at 25°C ± 1°C) and (ii) a pH of from 4.0 to 10, particularly a pH of from 6.0 to 8.0.

Typically, compositions of the invention are stable for at least 3 months at a temperature of 25°C, particularly for at least 6 months at a temperature of 25°C, more particularly for at least 12 months at a temperature of 25°C, yet more particularly for at least 24 months at a temperature of 25°C and still yet more particularly for 36 months at a temperature of 25°C. As used herein, the term "stable" refers to the ability of the composition to maintain certain physical or chemical properties, such as adhesion, at a specific storage temperature for a period of time after production. The stability of compositions of the present invention may be assessed using standard methods known in the art.

In certain embodiments, denture adhesive compositions do not contain silica, titanium dioxide, polyethylene glycol, sodium alginate, zinc or combinations thereof. In certain embodiments, denture adhesive compositions are free of silica, titanium dioxide, polyethylene glycol, sodium alginate, zinc or combinations thereof. In certain embodiments, denture adhesive compositions of the Invention are free of di-basic sodium phosphate, tetra sodium pyro phosphate and sodium tri poly phosphate.

Thus, in some embodiments the denture adhesive composition is a zinc-free denture adhesive composition. In some embodiments the denture adhesive composition is a titanium dioxide free denture adhesive composition. In some embodiments the denture adhesive composition is a silica-free denture adhesive composition.

In certain embodiments of the Invention, the denture adhesive composition consists essentially of: (a) from 28-32% w/w of poly(methylvinylether/maleic acid) Sodium-Calcium Mixed Partial Salt; (b) from 23-25% w/w of sodium carboxymethylcellulose; (c) from 18-25% w/w of medium viscosity mineral oil; (d) from 18%-26% w/w of petrolatum; (e) 1.5-2.5% w/w of *Helianthus annuus* (sunflower) seed oil comprising from 1% to 5% of an *aloe barbadensis* extract; and optionally (f) 0.1% w/w of an aloe flavour agent. Particularly following preparation such compositions have a viscosity of from 300,000 to 2,000,000 centipoise (measured at 25°C ± 1°C), more particularly of from 400,000 to 1,000,000 centipoise (measured at 25°C ± 1°C), or yet more particularly of from 400,000 to 500,000 centipoise (measured at 25°C ± 1°C) and a pH of from 5.0 to 8.0, more particularly a pH of from 6.0 to 8.0.

There is also provided a denture adhesive composition of the Invention for use in a method of adhering a dental appliance to a surface, particularly a surface in the oral cavity. There is also provided a method of adhering a dental appliance to a surface, particularly a surface in the oral cavity, using a denture adhesive composition of the Invention. There is also provided a denture adhesive composition of the Invention for use in a method of improving food seal of a dental appliance. There is also provided a denture adhesive composition of the Invention for use in a method of preventing or reducing ingress of food particles from a cavity, particularly the oral cavity of a subject, into the interstice between a dental appliance and the palate or gum ridge of the subject.

Particularly, the methods may comprise applying a composition of the First Aspect to a dental appliance, the oral cavity, or both, and applying the dental appliance to a surface of the oral cavity, such as the palate or a gum ridge.

There is also provided a denture adhesive composition of the Invention for use in a method of improving, for example increasing, food seal of a dental appliance comprising applying a composition of the Invention to a dental appliance, the oral cavity, or both, and applying the dental appliance to a surface of the oral cavity, such as the palate or a gum ridge. There is also provided a method of improving, for example increasing, food seal of a dental appliance comprising applying a composition of the Invention to a dental appliance, the oral cavity, or both, and applying the dental appliance to a surface of the oral cavity, such as the palate or a gum ridge.

There is also provided a denture adhesive composition of the Invention for use in a method of preventing or reducing ingress of food particles from a cavity, particularly the oral cavity of a subject, into the interstice between a dental appliance and the palate or gum ridge of the subject comprising applying a composition of the Invention to a dental appliance, the oral cavity, or both, and applying the dental appliance to a surface of the oral cavity of the subject, such as the palate or a gum ridge. There is also provided a method of preventing or reducing ingress of food particles from a cavity, particularly the oral cavity of a subject, into the interstice between a dental appliance and the palate or gum ridge of the subject comprising applying a composition of the Invention to a dental appliance, the oral cavity, or both, and applying the dental appliance to a surface of the oral cavity of the subject, such as the palate or a gum ridge.

Food seal refers to providing a seal along the edges of a dental appliance to reduce or prevent ingress of food particles, thereby avoiding food becoming trapped between the dental appliance and the palate or gum ridge where it can cause irritation to the dental appliance wearer. Reference to improving food seal of a dental appliance includes preventing, delaying, and/or reducing the ability of food particles to enter the spaces between a dental appliance and the palate or gum ridge. Food seal or block may be assessed using tests known in the art, more particularly food-occlusion testing performed by assessing peanut particle migration under dentures. Thus, improvements in food seal may be assessed using food-occlusion testing comparing particle migration under dentures with and without the use of denture adhesive. Particularly, using quantitative testing, the use of the compositions of the Invention result in a reduction in the mass of peanut particles found under the denture when compared to a control, for example comparing dentures with and without an adhesive. Improvements in food seal may also be assessed by qualitative testing in patient trials with and without denture adhesive where participants are asked to rate the quantity of particles they perceive as being under their dentures. Particularly, the use of the compositions of the Invention result in a reduction in the number of participants aware of food particles under their dentures.

### General

The term "comprising" encompasses "including" e.g. a composition "comprising" X may include something additional e.g. X + Y. In some implementations, the term "comprising" refers to the inclusion of the indicated active agent, such as recited compounds, as well as inclusion of other active agents, and carriers, excipients, emollients, stabilizers, etc., known in the consumer health industry and generally recognized as safe (GRAS). Use of the transitional phrase "consisting essentially of" means that the scope of a claim is to be interpreted to encompass the specified materials or steps recited in the claim, and those that do not materially affect the basic and novel characteristic(s) of the claimed invention. See, In re Herz, 537 F.2d 549, 551-52, 190 USPQ 461, 463 (CCPA 1976) (emphasis in the original); see also MPEP 9 2111.03. Thus, the term "consisting essentially of" when used in a claim of this invention is not intended to be interpreted to be equivalent to "comprising". The term "consisting of" and variations thereof means including and limited to (for example, the specific recited constituents or steps). In certain territories, the term "comprising an active ingredient consisting of" may be used in place of "consisting essentially". The term "about" in relation to a numerical value x is optional and means, for example, that the numerical value may comprise some variance around the stated number to allow for routine experimental fluctuation, measurement variance or to encompass minor deviations that may achieve substantially the same results as the stated number, such as x±10%, x±5%, x±4%, x±3%, x±2% or x±1%. The word "substantially" does not exclude "completely" e.g. a composition which is "substantially free" from Y may be completely free from Y. Where necessary, the word "substantially" may be omitted from the definition of the invention. All percentages and ratios used herein are by weight of total composition, unless otherwise indicated.

All references or patent applications cited within this patent specification are incorporated by reference herein.

### Embodiments of the Invention

The following clauses describe certain specific embodiments of the invention:
**EMBODIMENT 1:** A denture adhesive composition comprising from about 18 to about 25 %w/w of medium viscosity mineral oil and an aloe component, wherein the composition has a viscosity of from 300,000-2,000,000 centipoise, particularly 300,000-500,000 centipoise.
**EMBODIMENT 2:** The denture adhesive composition of EMBODIMENT 1 comprising: (a) from about 18 to about 25 %w/w of medium viscosity mineral oil, (b) an *aloe* component, (c) an alkyl vinyl ether-maleic acid copolymer or salt thereof, (d) petrolatum and (e) carboxymethylcellulose, wherein the composition has a viscosity of from 300,000-1,000,000 centipoise, particularly 300,000-500,000 centipoise.
**EMBODIMENT 3:** The denture adhesive composition of EMBODIMENT 1 or EMBODIMENT 2 comprising: (a) from about 18 to about 25 %w/w of medium viscosity mineral oil, (b) an *aloe* component, (c) from about 28 to about 32 percent by weight (%w/w) of an alkyl vinyl ether-maleic acid copolymer or salt thereof, (d) from about 18 to about 26 %w/w of petrolatum and (e) from about 23 to about 25 %w/w of carboxymethylcellulose, wherein the composition has a viscosity of from 300,000-1,000,000 centipoise, particularly 300,000-500,000 centipoise.
**EMBODIMENT 4:** The denture adhesive composition of EMBODIMENT 1, 2 or 3 comprising from about 0.02 to about 2.5 %w/w of the aloe component.
**EMBODIMENT 5:** The denture adhesive composition of EMBODIMENT 4, wherein the aloe component is selected from the group consisting of an aloe extract, a powdered aloe extract, an aloe extract in oil or combination thereof.
**EMBODIMENT 6:** The denture adhesive composition of any one of EMBODIMENTS 1 to 5 further comprising at least one sensory component, for example a flavour component, fragrance, colorant or mixture thereof.
**EMBODIMENT 7:** The denture adhesive composition of any one of EMBODIMENTS 1 to 6 wherein the composition has a viscosity of from 400,000 to 500,000 centipoise (cP).
**EMBODIMENT 8:** The denture adhesive composition of EMBODIMENT 7 wherein the composition has a pH of from 5.0 to 8.0, particularly a pH of from 6.0 to 7.5.
**EMBODIMENT 9:** A denture adhesive composition comprising or consisting essentially of: The denture adhesive composition of any preceding claim which comprises or consists essentially of: (a) from 28-32% w/w of poly(methylvinylether/maleic acid) Sodium-Calcium Mixed Partial Salt; (b) from 23-25% w/w of sodium carboxymethylcellulose; (c) from 18-25% w/w of medium viscosity mineral oil; (d) from 18%-26% w/w of petrolatum; (e) 1.5-2.5% w/w of *Helianthus annuus* (sunflower) seed oil comprising from 1% to 5% of an *aloe barbadensis* extract; and optionally (f) 0.1% w/w of an aloe flavour agent.
**EMBODIMENT 10:** The denture adhesive composition of EMBODIMENT 9, wherein the composition has a viscosity of from 300,000 to 1,000,000 centipoise (cP) and a pH of from 6.0 to 8.0.

In order that this invention may be better understood, the following examples are set forth. These examples are for purposes of illustration only and are not to be construed as limiting the scope of the invention in any manner.

### EXAMPLES

### Example 1 - Denture Adhesive Formulations I

Six formulations, comprising varying amounts (0.1-5% w/w) of spray dried Aloe vera gel powder, were prepared using commercially available ingredients:

| | **1** | **2** | **3** | **4** | **3** | **6** |
|---|---|---|---|---|---|---|
| **Poly(Methylvinylether/maleic acid) Sodium-Calcium Mixed Partial Salt (Polisalt)** | 30 | 30 | 30 | 30 | 30 | 30 |
| **Sodium Carboxymethyl Cellulose (CMC)** | 24 | 24 | 24 | 24 | 24 | 24 |
| **Mineral Oil Medium** | 25 | 25 | 25 | 25 | 25 | 25 |
| **Petrolatum Blend** - **Pionier 6147** | 20.9 | 20.7 | 20.5 | 20 | 18 | 16 |
| **Aloe Vera Gel Spray dried powder 200x Enhanced organic 06-7123 (Concentrated aloe corp)** | 0.1 | 0.3 | 0.5 | 1 | 3 | 5 |

Physical and visual testing was performed using standard techniques.

Adhesive strength was determined by testing using standard methods as described in ISO-10873.

Work of adhesion was determined using an Instron Texture Analyzer (5943) equipped with automated spray and a 500 N load cell (Instron, UK). A quantity of adhesive was placed onto the test surface of the texture analyser, compressed to a uniform thickness of 1mm across the test surface and then taken through a number of cycles of compression/stretching. Following the compression/stretching cycles, the cross head was rapidly moved apart with speed of 6lbf/min and the maximum tensile force recorded. This was repeated at least 20 times for each sample.

Initial testing confirmed the viability of adding Aloe vera gel powder to denture adhesive formulations:

| | **1** | **2** | **3** | **4** | **5** | **6** |
|---|---|---|---|---|---|---|
| Appearance | Homogeneou s smooth paste | Homogeneou s smooth paste | Homogeneou s smooth paste | Homogeneou s smooth paste | Homogeneou s smooth paste | Homogeneou s smooth paste |
| Extrudabilit y | Easy | Easy | Easy | Easy | Easy to moderate | Easy to moderate |
| Separation | No Syneresis | No Syneresis | No Syneresis | No Syneresis | No Syneresis | No Syneresis |
| pH | 7.073 | 7.007 | 6.989 | 7.078 | 7.060 | 7.004 |
| Adhesive Strength: Test ISO-10873 (kPa) | 51.895 | 50.076 | 48.271 | 50.220 | 47.913 | 47.038 |
| Shear strength (lbf) | 2.866 | 2.963 | 3.074 | 2.514 | 2.025 | 2.384 |
| Viscosity (cPs) | 680,400 | 670,800 | 681,600 | 635,200 | 804,800 | 1,052,000 |
| Instron spray work of adhesion (J/m²) | 222 | 363 | 383 | 291 | 387 | 372 |

Thus, aloe vera powder was demonstrated to be compatible with the denture adhesive base formulation. The results also indicated a trend towards higher viscosity as the content of aloe vera powder was increased.

### Example 2 - Denture Adhesive Formulations II

In order to further reduce viscosity, additional formulations were prepared using a preparation of Aloe vera in oil and differing proportions of mineral oil and petrolatum:

| | **7** | **8** | **9** | **10** | **11** |
|---|---|---|---|---|---|
| **Poly(Methylvinylether/maleic acid) Sodium-Calcium Mixed Partial Salt (Polisalt)** | 30 | 30 | 30 | 30 | 30 |
| **Sodium Carboxymethyl Cellulose (CMC)** | 24 | 24 | 24 | 24 | 24 |
| **Mineral Oil Medium** | 7 | 20 | 21 | 22 | 23 |
| **Petrolatum Blend - Pionier 6147** | 29 | 24 | 23 | 22 | 21 |
| **Phytoconcentrole Aloe Vera S (447359)** | 10 | 2 | 2 | 2 | 2 |

Testing confirmed the viability of adding an Aloe vera oil preparation to a denture adhesive formulation:

| | **7** | **8** | **9** | **10** | **11** |
|---|---|---|---|---|---|
| **Appearance** | Homogeneous, smooth paste | Homogeneous, smooth paste | Homogeneous, smooth paste | Homogeneous, smooth paste | Homogeneous, smooth paste |
| **Extrudability** | Easy | Easy | Easy | Easy | Easy |
| **Separation** | No Syneresis | No Syneresis | No Syneresis | No Syneresis | No Syneresis |
| **pH** | 6.838 | 7.144 | 7.087 | 7.123 | 7.047 |
| **Adhesive Strength** - **10 mins (Kpa)** | 46.884 | 44.663 | 44.084 | 46.25 | 49.135 |
| **Instron spray work of adhesion** (J/m²) | 369 | 367 | 359 | 318 | 267 |
| **Viscosity (cPs)** | 785,600 | 484,000 | 433,600 | 388,400 | 307,00 |

However, some formulations - for example 11 - exhibited reduced hold, as evidenced by work of adhesion.

### Example 3 - Denture Adhesive Formulations III

Four further formulations were prepared and compared to investigate the inclusion of colloidal silicon dioxide (a constituent often used to reduce syneresis and oil separation) and to compare different aloe vera preparations:

| | **12** | **13** | **14** | **15** |
|---|---|---|---|---|
| Poly(Methylvinylether/maleic acid) Sodium-Calcium Mixed Partial Salt (Polisalt) | 30 | 30 | 30 | 30 |
| Carboxymethyl Cellulose (CMC) | 24 | 24 | 24 | 24 |
| Mineral oil (Medium) | 25 | 25 | 25 | 20 |
| Petrolatum (6147) | 20.5 | 20.4 | 18 | 24 |
| Colloidal silicon dioxide | 0.5 | 0.5 | 1.0 | - |
| Aloe vera Gel PE 5% MPS 200:1 (Naturex powder) | - | 0.1 | - | - |
| Phytoconcentrole Aloe Vera S (Symrise oil) | - | - | 2 | 2 |

Appearance, viscosity and adhesive strength were tested for each formulation:

| | **12** | **13** | **14** | **15** |
|---|---|---|---|---|
| **Appearance** | Homogeneous, smooth paste | Homogeneous, smooth paste | Homogeneous, smooth paste | Homogeneous, smooth paste |
| **Extrudability** | Easy | Easy/Moderate | Easy | Easy |
| **Separation** | Negligible/Slight | Negligible/Slight | No | Negligible/Slight |
| **pH** | 7.4 | 7.4 | 6.966 | 7.3 |
| **Adhesive Strength** - **10 mins (Kpa)** | 51 | 51 | 45.5 | 52 |
| **Instron spray work of adhesion** (J/m²) | 444 | 441 | 258 | 367 |
| **Viscosity (cPs)** | 967,200 | 788,400 | 335,600 | 599,200 |

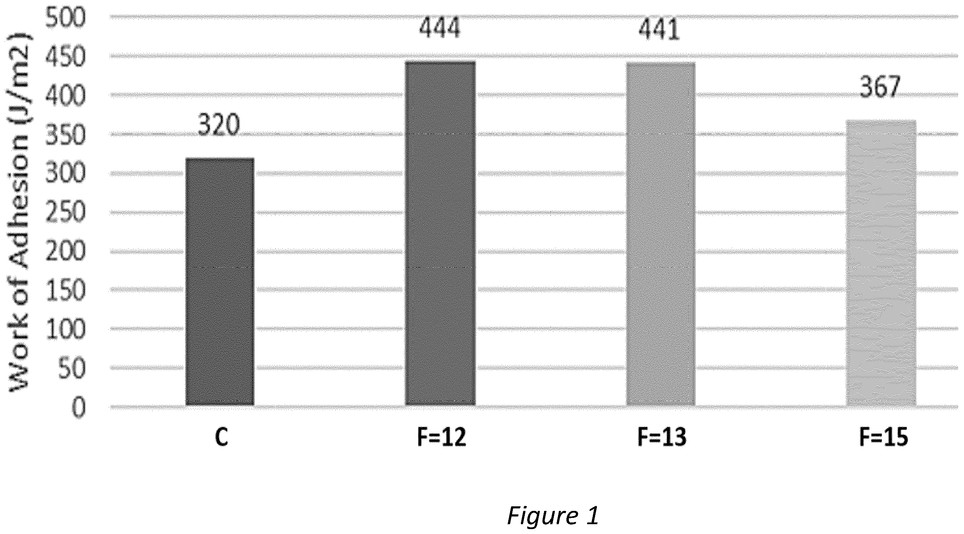

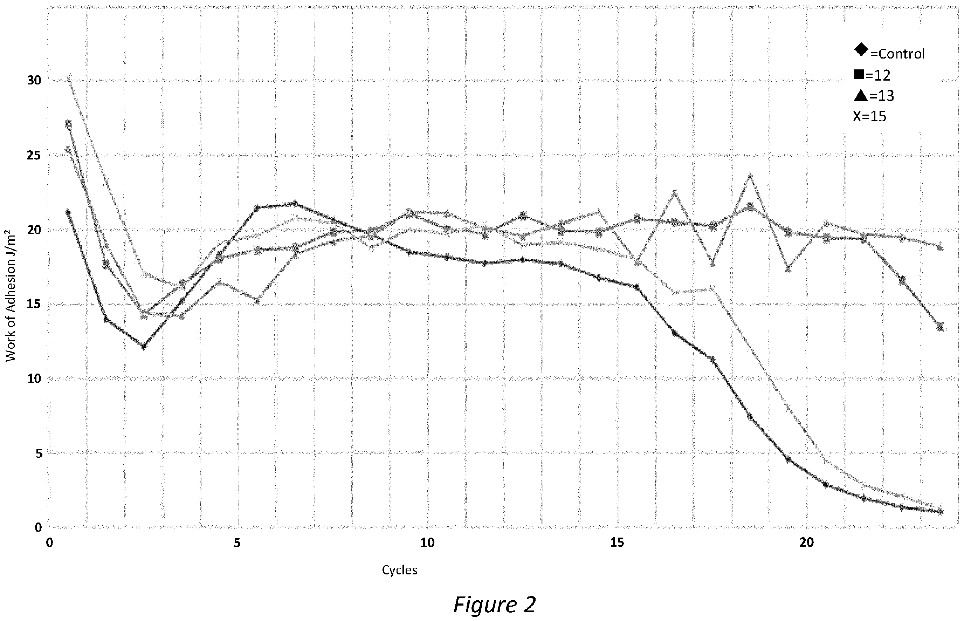

The addition of silica helped to reduce syneresis and oil separation in the high oil bases but impacted the viscosity and adhesion in higher amounts. Given the low work of adhesion, formulation 14 was not tested further. Figure 1 provides a bar chart of Instron work of adhesion values for formulations 12, 13 and 15 compared to a commercially available control (Poligrip Max Hold & Seal). Figure 2 provides a graph of Instron work of adhesion values over time for formulations 12, 13 and 15 compared to a commercially available control (Poligrip Max Hold & Seal). Surprisingly, whilst formulations 12 and 13 appeared to show relatively consistent hold over time, in confidential controlled patient testing, there was a clear preference for formulation 15. This was because formulations 12 and 13 were more difficult to remove at the end of the test, often leaving a sticky residue in the mouth.

### Example 4 - Denture Adhesive Formulations IV

Based on the above formulations and learnings, two further formulations were generated:

| | **16** | **17** |
|---|---|---|
| **Poly(Methylvinylether/maleic acid) Sodium-Calcium Mixed Partial Salt (Polisalt)** | 30 | 30 |
| **Carboxymethyl Cellulose (CMC)** | 24 | 24 |
| **Mineral oil (Medium)** | 19.9 | 21.9 |
| **Petrolatum (6147)** | 24 | 22 |
| **Phytoconcentrole Aloe Vera S (Symrise oil)** | 2 | 2 |
| **Micellar Aloe Flavour** | 0.1 | 0.1 |

Appearance, viscosity and adhesive strength were tested for each formulation:

| | **16** | **17** |
|---|---|---|
| **Appearance** | Homogenous smooth paste | Homogenous smooth paste |
| **Extrudability** | Easy/Moderate | Easy |
| **Separation** | No | No |
| **pH** | 7.145 | 7.146 |
| **Adhesive Strength** - **10 mins (Kpa)** | 44.612 | 43.928 |
| **Instron spray work of adhesion** (J/m²) | 357 | 351 |
| **Viscosity (cPs)** | 484,800 | 412,400 |

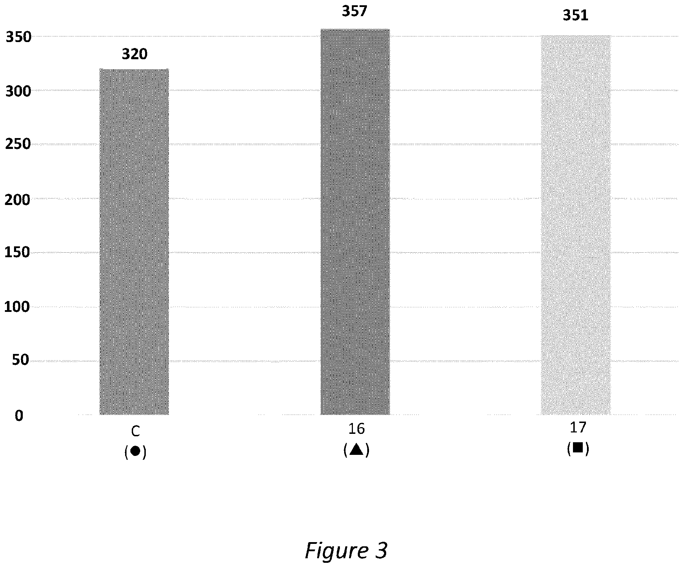

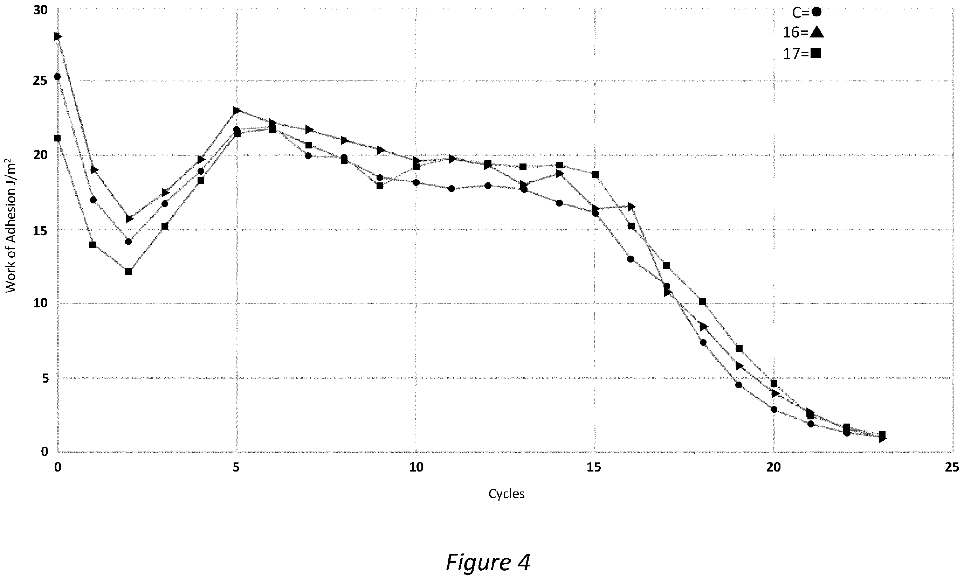

These two formulations demonstrated reduced viscosities, exhibiting higher levels of adhesion than the commercially marketed formulation (Figure 3) with an equivalent hold-profile over time (Figure 4) matching consumer preferences.

The purpose of the above description is to illustrate some embodiments of the present invention without implying a limitation. It will be apparent to those skilled in the art that various modifications and variations may be made in the apparatus or procedure of the invention without departing from the scope or spirit of the invention.

## Claims

1. A denture adhesive composition comprising (a) from about 18 to about 25 %w/w of medium viscosity mineral oil and (b) an *aloe* component, wherein the composition has a viscosity of from 300,000-2,000,000 centipoise ((cP) measured at 25°C ± 1°C).

2. The denture adhesive composition of claim 1 comprising: (a) from about 18 to about 25 %w/w of medium viscosity mineral oil, (b) an *aloe* component, (c) an alkyl vinyl ether-maleic acid copolymer or salt thereof, (d) petrolatum and (e) carboxymethylcellulose.

3. The denture adhesive composition of claim 2 comprising: (a) from about 18 to about 25 %w/w of medium viscosity mineral oil, (b) an *aloe* component, (c) from about 28 to about 32 percent by weight (%w/w) of an alkyl vinyl ether-maleic acid copolymer or salt thereof, (d) from about 18 to about 26 %w/w of petrolatum and (e) from about 23 to about 25 %w/w of carboxymethylcellulose, wherein the composition has a viscosity of from 300,000-2,000,000 centipoise.

4. The denture adhesive composition of any one of claims 1 to 3, comprising from about 0.02 to about 2.5 %w/w of the *aloe* component.

5. The denture adhesive composition of claim 4, wherein the aloe component is selected from the group consisting of a powdered aloe extract, a concentrated gel preparation, an aloe extract in oil or combinations thereof.

6. The denture adhesive composition of claim 5, wherein the aloe component is an aloe extract in oil.

7. The denture adhesive of Claim 6, wherein the aloe extract in oil is an aloe extract in a plant seed oil or a mineral oil.

8. The denture adhesive of Claim 7, wherein the plant seed oil is *Helianthus annuus*(*sunflower*) seed oil.

9. The denture adhesive of Claim 8, wherein the aloe component comprises from about 1.5 to about 2.5 %w/w of *Helianthus annuus* (*sunflower*) seed oil comprising from 1% to 5% of an *aloe barbadensis* extract.

10. The denture adhesive of Claim 9, wherein the aloe component further comprises an aloe flavour agent or a powdered aloe extract.

11. The denture adhesive of Claim 10, comprising 0.1 %w/w of aloe flavour agent or a powdered aloe extract.

12. The denture adhesive composition of any preceding claim, wherein the alkyl vinyl ether-maleic acid copolymer or salt thereof is a poly(methylvinylether/maleic acid) salt particularly a poly(methylvinylether/maleic acid) Sodium-Calcium Mixed Partial Salt.

13. The denture adhesive composition of Claim 12, wherein the carboxymethylcellulose has a viscosity of about 1000 to about 2800 centipoise (cP) (measured in a 1% solution at 25°C).

14. The denture adhesive composition of Claim 13, wherein the carboxymethylcellulose consists of fine particles of which at least 80% pass through a 200 U.S. mesh sieve (equivalent to a 74 µm mesh sieve).

15. The denture adhesive composition of any one of Claim 13 or 14, wherein the carboxymethylcellulose is sodium carboxymethylcellulose optionally having a degree of substitution (DS) of from 0.65 to 0.95, particularly about 0.7.

16. The denture adhesive composition of any one of Claims 10 to 13, wherein the composition has a pH of from 5.0 to 8.0, particularly a pH of from 6.0 to 8.0.

17. The denture adhesive composition of any preceding claim which comprises or consists essentially of:
(a) from 28-32% w/w of poly(methylvinylether/maleic acid) Sodium-Calcium Mixed Partial Salt;
(b) from 23-25% w/w of sodium carboxymethylcellulose;
(c) from 18-25% w/w of medium viscosity mineral oil;
(d) from 18%-26% w/w of petrolatum;
(e) 1.5-2.5% w/w of *Helianthus annuus* (sunflower) seed oil comprising from 1% to 5% of an *aloe barbadensis* extract; and optionally
(f) 0.1% w/w of an aloe flavour agent or powdered aloe extract.

18. The denture adhesive composition of Claim 17, wherein the composition has a viscosity of from 300,000 to 500,000 centipoise (measured at 25°C ± 1°C) and a pH of from 6.0 to 8.0.

19. The denture adhesive of Claim 17 or 18, wherein when applied to a support surface (carrier) the denture adhesive exhibits total work of adhesion values (W_{AUC}) of at least 300 J/m² in an Instron based in vitro adhesion model.
